**Europäisches Patentamt**

**European Patent Office**

**Office européen des brevets**

(11) Veröffentlichungsnummer : **0 282 453 B1**

(12) **EUROPÄISCHE PATENTSCHRIFT**

(45) Veröffentlichungstag der Patentschrift :
**06.05.92 Patentblatt 92/19**

(51) Int. Cl.$^5$ : **A61K 31/785, A61K 47/00**

(21) Anmeldenummer : **88810134.2**

(22) Anmeldetag : **04.03.88**

(54) **Hypocholesterinämische Gelformulierung auf Anionenaustauscherharz-Basis.**

(30) Priorität : **10.03.87 US 24225**
**10.03.87 US 24224**

(43) Veröffentlichungstag der Anmeldung :
**14.09.88 Patentblatt 88/37**

(45) Bekanntmachung des Hinweises auf die
Patenterteilung :
**06.05.92 Patentblatt 92/19**

(84) Benannte Vertragsstaaten :
**AT BE CH DE ES FR GB GR IT LI LU NL SE**

(56) Entgegenhaltungen :
**EP-A- 0 190 826**
**FR-A- 2 110 458**
**US-A- 3 974 272**

(73) Patentinhaber : **CIBA-GEIGY AG**
**Klybeckstrasse 141**
**CH-4002 Basel (CH)**

(72) Erfinder : **Dechow, Frederick J.**
**P.O. Box 1300**
**Summit New Jersey 07901 (US)**

## Beschreibung

Pharmazeutisch verwendbare Polystyrol-Anionenaustauscherharze mit schwacher Vernetzung und quaternären Ammoniumgruppen, z.B. Cholestyraminharz in Form von Pulver, sind als hypocholesterinämische Mittel bekannt, z.B. U.S. Patentschrift 3,383,281 (Wolf et al.).

Solche Harze werden oral appliziert, z.B. durch Zumischen des Pulvers mit einem Getränk wie Wasser, Milch, Fruchtsaft oder einem anderen Getränk ohne Kohlensäuregehalt, in Form von Suspensionen, z.B. durch Aufschlämmen mit Fruchtfleisch mit hohem Wassergehalt, z.B. Apfelsirup oder zerstossener Ananas. Bei der Passage durch den gastrointestinalen Trakt adsorbiert das nicht-metabolisierbare Harz in seiner Funktion als Ionenaustauscher Cholsäurederivate, welche als unlösliches Assoziat ausgeschieden werden. Durch die Entfernung von Cholsäurederivaten wird die Oxydation von Cholesterin zu Cholsäurederivaten angeregt, was eine Abnahme im Beta-Lipoproteinspiegel, insbesondere einen geringeren Gehalt von Lipoprotein im Plasma, und generell eine Abnahme des Serumcholesterinspiegels bewirkt.

Die genannten Harze sind folglich bei der Minderung von erhöhtem Cholesterinspiegel in Patienten mit hypocholesterinämischem Befund und bei der Behandlung von Pruritus verwendbar.

Die genannten Harze sind aufgrund ihrer äusserst unangenehmen geschmacklichen Eigenschaften bei der Einnahme trotz Maskierung des Geschmacks durch Fruchtaroma in den genannten Getränken und Zubereitungen nachteilig. Bei einigen Patienten können sogar Schluckbeschwerden auftreten, was zu mangelnder Akzeptanz und einem Unterlassen der Einnahme führen kann.

In der Europäischen Patentanmeldung 190 826 sind Formulierungen für Polystyrol-Anionenaustauscherharze beschrieben, die zur Verbesserung des Geschmacks als wesentliche Bestandteile Hydrocolloide auf Kohlehydratbasis wie Karayagummi enthalten.

Der vorliegenden Erfindung liegt die Aufgabe zugrunde, eine pharmazeutisch verwendbare, halbfeste Zusammensetzung mit einer hypocholesterinämisch wirksamen Menge eines pulverförmigen Polystyrol-Anionenaustauscherharzes mit schwacher Vernetzung und quaternären Ammoniumgruppen mit guten geschmacklichen Eigenschaften und gegenüber Hydrocolloiden verbesserter Festigkeit herzustellen.

Der vorliegenden Erfindung liegt ausserdem die Aufgabe zugrunde, ein Herstellungsverfahren für die genannte pharmazeutische Zusammensetzung bereitzustellen, unter Verwendung der für diese Zusammensetzung erforderlichen Komponenten. Ebenfalls Gegenstand der Erfindung ist die therapeutische Verwendung der pharmazeutischen Zusammensetzung, insbesondere als hypocholesterinämisches Mittel.

Diese Aufgaben werden durch die vorliegende Erfindung gelöst, welche eine halbfeste Formulierung mit hypocholesterinämischer Wirkung für die orale Anwendung zum Gegenstand hat. Die Formulierung enthält eine hypocholesterinämisch wirksame Menge einer einheitlich gelförmigen Dispersion mit folgenden Komponenten:

(a) 8 bis 20 Gew.-% eines pharmazeutisch annehmbaren Polystyrol-Anionenaustauscherharzes mit schwacher Vernetzung und quaternären Ammoniumgruppen in feiner Verteilung mit einer durchschnittlichen Teilchengrösse unterhalb ca. 100 Mikrometer;

(b) 1,5 bis 16 Gew.-% Gelatine;

(c) 0.015 bis 10 Gew.-% eines pharmazeutisch annehmbaren, natürlichen oder synthetischen Süßstoffs;

(d) 0,05 bis 2 Gew.-% eines pharmazeutisch annehmbaren Säuerungsmittels;

(e) 0,05 bis 5 Gew.-% eines pharmazeutisch annehmbaren Geschmacksoder Farbstoffs oder Mischungen davon und gegebenenfalls 0,01-1 Gew.-% pharmazeutisch annehmbare Konservierungsmittel, Stabilisatoren und Quellmittel; und

(f) Wasser ad 100 %.

Die Anionenaustauscherharz-Komponente (a), welche in feiner Verteilung vorliegt, gehört zu einer Gruppe von Anionenaustauscherharzen, welche für die Senkung des Cholesterinspiegels und des Triglyceridspiegels verwendbar sind, sowie für die Behandlung von Pruritus geeignet sind. Solche Harze besitzen quaternäre Ammoniumgruppen am Polystyrol-Grundgerüst. Bevorzugt ist das Polystyrol-Grundgerüst durch Tri-niederalkylammoniumgruppen, insbesondere durch Trimethylammoniumgruppen, jeweils an der Phenylgruppe durch Bindung an ein Stickstoffatom substituiert. Das Harz ist nur schwach vernetzt, insbesondere durch die üblichen Divinylgruppen, vor allem Divinylbenzol.

Der Gehalt an Netzmittel beträgt weniger als 5 %, insbesondere 1 bis 4 %, vor allem 2 Gew.-% bezogen auf das Trockengewicht des Harzes. Geeignete Harze können nach den in der U.S. Patentschrift 2,591,573 gegebenen Vorschriften hergestellt werden. Die quaternäre Ammoniumgruppe wird durch das Anion einer pharmazeutisch annehmbaren Säure, z.B. durch Chlorid-, Acetat- oder Sulfatanionen abgesättigt. Das Anionenaustauscherharz hat vorzugsweise einen Wassergehalt von mehr als 65 % (nach Stehenlassen an der Luft bei 20°C und 100 % relativer Luftfeuchte). Insbesondere ist das in der U.S. Pharmakopoe (U.S.P.) beschriebene Cholestyramin-Harz bevorzugt. Das Harz soll so mikronisiert sein, dass mehr als 80 % der Teilchenmenge in einer durchschnittlichen Teilchengrösse von weniger als 100 Mikrometer vorliegen. Vorzugsweise haben mindestens 80 Prozent der Teilchenmenge eine durchschnittliche Teilchengrösse zwischen 20 und 100 Mikrometer und weniger als 0,5 % eine Teilchengrösse grösser als 200 Mikrometer. Der bevorzugte Mengenanteil der Komponente (a) in der erfindungsgemässen Zusammensetzung beträgt 12 bis 18 Gew.-%.

Die Komponente (b) besteht aus pharmazeutisch annehmbarer Gelatine, z.B. Gelatine mit U.S.P. Qualität mit Löslichkeit in heissem Wasser oberhalb 35°C. Die Komponente ist in einer bevorzugten Menge von 4-16 % in der Zusammensetzung enthalten und bildet in dieser Menge eine formstabile, elastische, kolloide Lösung, worin das Anionenaustauscherharz (a) beim Abkühlen bis auf 20°C gleichmässig dispergiert wird. Ein besonders bevorzugter Mengenbereich beträgt 5 bis 10 Gew.-% bezogen auf das Gesamtgewicht der Formulierung. Insbesondere ist Gelatine U.S.P. Typ A bevorzugt. Die Gelatine dient ausserdem zur Maskierung der vom Anionenaustauscherharz (a) ausgehenden unangenehmen Geschmackseigenschaften.

Das pharmazeutisch annehmbare Säuerungsmittel (d) ist in der Zusammensetzung mit den Süßstoffen (c) und der Gelatine (b) enthalten, um ebenfalls die nachteiligen Geschmackseigenschaften zu überdecken. Geeignet sind beispielsweise pharmazeutisch unbedenkliche Säuren wie Adipinsäure, Ascorbinsäure, Citronensäure, Apfelsäure, Weinsäure oder Mischungen davon. Die besonders bevorzugten Mengenanteile variieren mit der verwendeten Säure. Generell ist der Bereich von 0,1 bis 0,5 Gewichtsprozent bezogen auf das Gesamtgewicht der Formulierung bevorzugt. Besonders bevorzugt ist Adipinsäure.

Pharmazeutisch annehmbare Geschmacks- und Farbstoffe (e) können aus der Fülle der in diversen Pharmakopöen angegebenen Stoffe ausgewählt werden und sind z.B. künstliche und natürliche Geschmacksstoffe wie Aromen von Limonen, Kirschen, Orangen, Bananen, grüner Minze, Zitrone, Himbeere, Blaubeere, Vanille, Erdbeere, Zimt, Pfefferminze u.ä.

Es können noch bei Bedarf geringe Mengen, inbesondere 0,01 bis 1 Gew.-%, pharmazeutisch annehmbare Konservierungsmittel, Stabilisatoren oder Quellmittel zugesetzt werden. Geeignete Konservierungsmittel sind z.B. Kaliumsorbat oder Natriumpropionat. Als Quellmittel ist Methylcellulose bei Gefahr von Obstipation verwendbar.

Die vorliegende Erfindung hat ebenfalls ein Verfahren zur Herstellung der weiter vorn beschriebenen pharmazeutischen Zusammensetzungen zum Gegenstand. Dieses Verfahren ist dadurch gekennzeichnet, dass man die Anionenaustauscherharz-Komponente (a) bis hin zu einer durchschnittlichen Teilchengrösse von weniger als 100 Mikrometer mikronisiert, eine wässrige Lösung der Gelatine (b) bei Temperaturen oberhalb 35°C herstellt und zu dieser wässrigen Lösung bei erhöhter oder erniedrigter Temperatur die Komponenten (a), (c), (d) und (e) hinzusetzt und anschliessend die gebildete Suspension abkühlen lässt.

Bei Verwendung der Gelatine kann vor dem Abkühlen und der Erstarrung des Gels die flüssige Dispersion mit dem Harz der Komponente (a) als Einheitsdosis in geeignete Formen oder Behälter geeigneter Grösse und Form gegossen werden, worauf die Dispersion mit dem Harz erhärtet, aber die gewünschte elastische Form beibehält. Alternativ kann die Gelbildung statt in Einheitsdosis in grösseren Behältern stattfinden, worauf man die Einheitsdosis durch Abtrennen geeigneter Portionen erhält.

Die Einheitsdosis ist variabel und von den Bedingungen der Behandlung abhängig. Bevorzugt werden 2 bis 16 g des Ionenaustauscherharzes, insbesondere 4 g, appliziert.

Die folgenden Beispiele illustrieren die Erfindung. Die Mengenangaben erfolgen in Gewichtsteilen.

Beispiel 1:

2,0 Gewichtsteile Gelatine U.S.P. Typ A werden in 24 Gewichtsteilen Wasser bei 60°C unter Rühren aufgelöst. Man rührt drei Minuten lang und gibt 4,0 Gewichtsteile trockenes Cholestyraminharz DOWEX 1 x 2 (Dow Chemical Midland Mich. USA), welches 2 % des Vernetzungsmittels Divinylbenzol enthält, hinzu. Das Harz ist zuvor durch Mikronisieren und Sieben auf eine durchschnittliche Teilchengrösse von weniger als 100 Mikrometer gebracht worden, wobei 80 Prozent eine durchschnittliche Teilchengrösse von 20 bis 100 Mikrometer haben. Bei 60° werden zu der Suspension noch 0,07 Gewichtsteile Aspartam hinzugesetzt. Man rührt noch einmal drei Minuten lang und setzt ebenfalls bei dieser Temperatur 0,10 Gewichtsteile Adipinsäure, 0,10 Gewichtsteile einer Mischung aus künstlichem Erdbeeraroma und FD&C Red (roter Farbstoff), gelöst in Aethanol, hinzu. Man rührt das Gemisch fünf Minuten lang und giesst die Suspension mit dem Anionenaustauscherharz und den übrigen Komponenten in feiner Verteilung in rechteckige Formen, worin, nach Abkühlen auf Raumtemperatur in einem Zeitraum von zwei Stunden, die elastische Formulierung als kolloidale Lösung oder Gel mit dem einheitlich suspendierten Harz darin erhalten wird.

Beispiel 2:

Man stellt eine pharmazeutische Zusammensetzung nach den in Beispiel 1 gegebenen Vorschriften her und fügt noch 0,2 Gewichtsteile Methylcellulose hinzu.

**Patentansprüche**

**Patentansprüche für folgende Vertragstaaten : AT, BE, CH, DE, FR, GB, IT,LI, LU, NL, SE**

1. Pharmazeutische Zusammensetzung in Form einer halbfesten Formulierung mit hypocholesterinämischer Wirkung bestehend aus einer oral anwendbaren Dispersion mit folgenden Komponenten:

(a) 8 bis 20 Gew.-% eines pharmazeutisch annehmbaren PolystyrolAnionenaustauscherharzes mit schwacher Vernetzung und quaternären Ammoniumgruppen in feiner Verteilung mit einer durchschnittlichen Teilchengrösse unterhalb 100 Mikrometer;

(b) 1,5 bis 16 Gew.-% Gelatine;

(c) 0,015 bis 10 Gew.-% eines pharmazeutisch annehmbaren, natürlichen oder synthetischen Süßstoffes;

(d) 0,05 bis 2 Gew.-% eines pharmazeutisch annehmbaren Säuerungsmittels;

(e) 0,05 bis 5 Gew.-% eines pharmazeutisch annehmbaren Geschmacksoder Farbstoffs oder Mischungen davon und gegebenenfalls 0.01 bis 1 Gew.-% pharmazeutisch annehmbare Konservierungsmittel, Stabilisatoren oder Quellmittel; und

(f) Wasser ad 100 %.

2. Pharmazeutische Zusammensetzung gemäss Anspruch 1, worin die Gelatine der Komponente (b) Gelatine U.S.P. Typ A ist.

3. Pharmazeutische Zusammensetzung gemäss Anspruch 1, worin der Süßstoff Aspartam und das Säuerungsmittel Adipinsäure ist.

4. Verfahren zur Herstellung von pharmazeutischen Zusammensetzungen gemäss Anspruch 1, dadurch gekennzeichnet, dass man die Anionenaustauscherharz-Komponente (a) bis zu einer durchschnittlichen Teilchengrösse von weniger als 100 Mikrometer mikronisiert, eine wässrige Lösung der Gelatine (b) bei Temperaturen oberhalb 35°C herstellt und zu dieser wässrigen Lösung bei erhöhter oder erniedrigter Temperatur die Komponenten (a), (c), (d) und (e) hinzusetzt und anschliessend die gebildete Suspension abkühlen lässt.

5. Pharmazeutische Zusammensetzung gemäss Anspruch 1 zur Behandlung des menschlichen oder tierischen Körpers.

6. Verwendung der Komponenten (a) bis (e) gemäss Anspruch 1 in einem Verfahren zur Herstellung von pharmazeutischen Zusammensetzungen.

**Patentansprüche für folgende Vertragstaaten : ES, GR**

1. Verfahren zur Herstellung einer pharmazeutischen Zusammensetzung in Form einer halbfesten Formulierung mit hypocholesterinämischer Wirkung bestehend aus einer oral anwendbaren Dispersion mit folgenden Komponenten:

(a) 8 bis 20 Gew.-% eines pharmazeutisch annehmbaren Polystyrol-Anionenaustauscherharzes mit schwacher Vernetzung und quaternären Ammoniumgruppen in feiner Verteilung mit einer durchschnittlichen Teilchengrösse unterhalb 100 Mikrometer;

(b) 1,5 bis 16 Gew.-% Gelatine;

(c) 0,015 bis 10 Gew.-% eines pharmazeutisch annehmbaren, natürlichen oder synthetischen Süßstoffs;

(d) 0,05 bis 2 Gew.-% eines pharmazeutisch annehmbaren Säuerungsmittels;

(e) 0,05 bis 5 Gew.-% eines pharmazeutisch annehmbaren Geschmacksoder Farbstoffs oder Mischungen davon und gegebenenfalls 0,01 bis 1 Gew.-% pharmazeutisch annehmbare Konservierungsmittel, Stabilisatoren oder Quellmittel; und

(f) Wasser ad 100 %,

dadurch gekennzeichnet, dass man das Anionenaustauscherharz der Komponente a) bis zu einer durchschnittlichen Teilchengrösse von weniger als 100 Mikrometer mikronisiert, eine wässrige Lösung der Gelatine (b) bei Temperaturen oberhalb 35°C herstellt und zu dieser wässrigen Lösung bei erhöhter oder erniedrigter Temperatur die Komponenten (a), (c), (d) und (e) hinzusetzt und anschliessend die gebildete Suspension abkühlen lässt.

2. Verfahren zur Herstellung einer pharmazeutischen Zusammensetzung gemäss Anspruch 1, worin die Gelatine der Komponente (b) Gelatine U.S.P Typ A ist.

3. Verfahren zur Herstellung einer pharmazeutischen Zusammensetzung gemäss Anspruch 1, worin der Süßstoff Aspartam und das Säuerungsmittel Adipinsäure ist.

## Claims

**Claims for the following Contracting States : AT, BE, CH, DE, FR, GB, IT,LI, LU, NL, SE**

1. A pharmaceutical composition in the form of a semi-solid formulation having hypocholesterolaemic activity and comprising an orally administrable dispersion comprising the following components:

(a) 8 to 20 percent by weight of a particulate sparingly cross-linked pharmaceutically acceptable quaternary ammonium substituted polystyrene anion exchange resin having an average particle size below 100 microns;

(b) 1.5 to 16 percent by weight of gelatin;

(c) 0.015 to 10 percent by weight of a natural or synthetic pharmaceutically acceptable sweetener;

(d) 0.05 to 2 percent by weight of a pharmaceutically acceptable acidifying agent;

(e) 0.05 to 5 percent by weight of a pharmaceutically acceptable flavouring or colouring agent or mixtures thereof, and, if necessary, 0.01 to 1 percent by weight of pharmaceutically acceptable preservatives, stabilisers or swelling agents; and

(f) water ad 100 %.

2. A pharmaceutical composition according to claim 1 wherein the gelatin component (b) is gelatin USP, type A.

3. A pharmaceutical composition according to claim 1 wherein the sweetener is aspartame and the acidifying agent is adipic acid.

4. A process for the preparation of a pharmaceutical composition according to claim 1, which comprises micronising the anion exchange resin component (a) to an average particle size of less than 100 microns, preparing an aqueous solution of the gelatin (b) at temperatures above 35°C and adding components (a), (c), (d) and (e) to that aqueoas solution at elevated or reduced temperature, and then allowing the resulting suspension to cool.

5. A pharmaceutical composition according to claim 1 for the treatment of the human or animal body.

6. The use of components (a) to (e) according to claim 1 in a process for the preparation of a pharmaceutical composition.

**Claims for the following Contracting States : ES, GR**

1. A process for the preparation of a pharmaceutical composition in the form of a semi-solid formulation having hypocholesterolaemic activity and comprising an orally administrable dispersion comprising the following components:

(a) 8 to 20 percent by weight of a particulate sparingly cross-linked pharmaceutically acceptable quaternary ammonium substituted polystyrene anion exchange resin having an average particle size below 100 microns;

(b) 1.5 to 16 percent by weight of gelatin;

(c) 0.015 to 10 percent by weight of a natural or synthetic pharmaceutically acceptable sweetener;

(d) 0.05 to 2 percent by weight of a pharmaceutically acceptable acidifying agent;

(e) 0.05 to 5 percent by weight of a pharmaceutically acceptable flavouring or colouring agent or mixtures thereof, and, if necessary, 0.01 to 1 percent by weight of pharmaceutically acceptable preservatives, stabilisers or swelling agents; and

(f) water ad 100 %,

which process comprises micronising the anion exchange resin component (a) to an average particle size of less than 100 microns, preparing an aqueous solution of the gelatin (b) at temperatures above 35°C and adding components (a), (c), (d) and (e) to that aqueous solution at elevated or reduced temperature, and then allowing the resulting suspension to cool.

2. A process for the preparation of a pharmaceutical composition according to claim 1 wherein the gelatin component (b) is gelatin USP, type A.

3. A process for the preparation of a pharmaceutical composition according to claim 1 wherein the sweetener is aspartame and the acidifying agent is adipic acid.

**Revendications**

**Revendications pour les Etats contractants suivants : AT, BE, CH, DE, FR, GB, IT, LI, LU, NL, SE**

1. Composition pharmaceutique sous forme d'une formulation semi-solide ayant une action hypocholestérolémiante, constituée par une dispersion utilisable de façon orale ayant les composants suivants :

a) de 8 à 20 % en poids d'une résine acceptable en pharmacie de polystyrène échangeuse d'anions, cette résine ayant une faible réticulation et des groupes d'ammonium quaternaire, en dispersion fine avec une dimension moyenne des particules inférieure à 100 micromètres;

b) de 1,5 à 16% en poids de gélatine;

c) de 0,015 à 10% en poids d'un édulcorant naturel ou synthétique acceptable en pharmacie;

d) de 0,05 à 2% en poids d'un agent d'acidification acceptable en pharmacie;

e) de 0,05 à 5% en poids d'un aromate ou d'une matière colorante acceptables en pharmacie, ou de leurs mélanges, et éventuellement de 0,01 à 1% en poids d'agents de conservation, de stabilisants ou d'agents gonflants, acceptables en pharmacie; et

f) de l'eau jusqu'à 100%.

2. Composition pharmaceutique selon la revendication 1, où la gélatine du composant (b) est de la gélatine U.S.P. de type A.

3. Composition pharmaceutique selon la revendication 1, où l'agent édulcorant est l'aspartame et l'agent d'acidification est l'acide adipique.

4. Procédé pour préparer des compositions pharmaceutiques selon la revendication 1, caractérisé en ce que l'on broie le composant (a) de résine échangeuse d'anions jusqu'à une dimension moyenne des particules inférieure à 100 micromètres, on prépare une solution aqueuse de la gélatine (b) à des températures supérieures à 35°C, et on ajoute à cette solution aqueuse, à une température supérieure ou inférieure, les composants (a), (c), (d) et (e), puis on laisse refroidir ensuite la suspension formée.

5. Composition pharmaceutique selon la revendication 1 pour traiter le corps humain ou le corps animal.

6. Utilisation des composants (a) à (e) selon la revendication 1 dans un procédé pour préparer des compositions pharmaceutiques.

**Revendications pour les Etats contractants suivants : ES, GR**

1. Procédé pour préparer une composition pharmaceutique sous forme d'une formulation semi-solide ayant une action hypocholestérolémiante, constituée par une dispersion utilisable de façon orale ayant les composants suivants

a) de 8 à 20% en poids d'une résine acceptable en pharmacie de polystyrène échangeuse d'anions, cette résine ayant une faible réticulation et des groupes d'ammonium quaternaire, en dispersion fine avec une dimension moyenne des particules inférieure à 100 micromètres;

b) de 1,5 à 16% en poids de gélatine;

c) de 0,015 à 10% en poids d'un édulcorant naturel ou synthétique acceptable en pharmacie;

d) de 0,05 à 2% en poids d'un agent d'acidification acceptable en pharmacie;

e) de 0,05 à 5% en poids d'un aromate ou d'une matière colorante acceptables en pharmacie, ou de leurs mélanges, et éventuellement de 0,01 à 1% en poids d'agents de conservation, de stabilisants ou d'agents gonflants, acceptables en pharmacie; et

f) de l'eau jusqu'à 100%,

caractérisé en ce que l'on broie la résine échangeuse d'anions du composant (a) jusqu'à une dimension moyenne des particules inférieure à 100 micromètres, on prépare une solution aqueuse de la gélatine (b) à des températures supérieures à 35°C, et on ajoute à cette solution aqueuse, à une température supérieure ou inférieure, les composants (a), (c), (d) et (e), puis on laisse refroidir ensuite la suspension formée.

2. Procédé pour préparer une composition pharmaceutique selon la revendication 1, où la gélatine du composant (b) est de la gélatine U.S.P. de type A.

3. Procédé pour préparer une composition pharmaceutique selon la revendication 1, où l'agent édulcorant est l'aspartame et l'agent d'acidification est l'acide adipique.